# EUROPÄISCHE PATENTANMELDUNG

(11) **EP 1 072 205 A2**
(43) Veröffentlichungstag der Anmeldung: **31.01.2001**
(21) Anmeldenummer: 00112128.4
(22) Anmeldetag: 06.06.2000
(51) Int. Cl.: A42B 3/24, A61F 9/02

(54) **Heizeinrichtung für einen Sichtkörper eines Schutzhelms oder einer Schutzbrille**

(30) Priorität: 02.07.1999 DE 19930603
(71) Anmelder: Bayerische Motoren Werke Aktiengesellschaft, 80809 München (DE)
(72) Erfinder: Madlener, Roland, 86949 Windach (DE); Borsos, Günter, 82216 Maisach (DE); Karg, Jochen, Dr., 85622 Feldkirchen (DE)

(57) **Zusammenfassung**

Es wird ein Sichtkörper (2) für einen Schutzhelm oder eine Schutzbrille beschrieben, mit einer aus elektrisch leitfähigem und mindestens teilweise durchsichtigem Material bestehenden Heizeinrichtung, die lösbar am Sichtkörper (2) angebracht ist. Zwischen einander gegenüberliegenden Oberflächen (8,9) des Sichtkörpers (2) und der Heizeinrichtung ist wenigstens über Teilbereiche von diesen ein Zwischenraum (7) vorhanden.

## Beschreibung

Die Erfindung bezieht sich auf eine Heizeinrichtung für einen Sichtkörper eines Schutzhelms oder einer Schutzbrille nach dem Oberbegriff des Anspruchs 1.

Es ist bereits bekannt, Sichtkörper für einen Schutzhelm oder eine Schutzbrille mit einer aus elektrisch leitfähigem und durchsichtigem Material bestehenden Heizeinrichtung zu versehen, wobei diese aus einer durchsichtigen Trägerfolie und darauf angeordnetem elektrisch leitfähigen Material besteht und einen lösbar am Sichtkörper befestigten Heizkörper bildet. Vorzugsweise kann dann der Heizkörper Ecken aufweisen, die in entsprechende Aufnahmen des Sichtkörpers einsetzbar sind. Desweiteren ist bekannt, daß der Heizkörper vorzugsweise auf der dem Benutzer zugewandten Seite des Sichtkörpers befestigt ist und daß das elektrisch leitfähige Material aus Heizdrähten besteht. Einen solchen Sichtkörper beschreibt die DE 32 29 021 C2.

Aufgabe der Erfindung ist es, einen solchen Sichtkörper für einen Schutzhelm oder eine Schutzbrille weiterzubilden, daß dieser eine noch bessere Sicherheit gegen Beschlagen besitzt, das heißt, gegen die Bildung eines Feuchtigkeitsfilms auf seiner Oberfläche.

Die Aufgabe wird erfindungsgemäß mit den Merkmalen des Anspruchs 1 gelöst. Weitere Ausgestaltungen der Erfindung ergeben sich aus den Unteransprüchen.

Nach der Erfindung ist ein Sichtkörper für einen Schutzhelm oder eine Schutzbrille, mit einer aus elektrisch leitfähigem und durchsichtigem Material bestehenden Heizeinrichtung, die lösbar am Sichtkörper angebracht ist, dadurch gekennzeichnet, daß zwischen einander gegenüberliegenden Oberflächen des Sichtkörpers und der Heizeinrichtung wenigstens über Teilbereiche von diesen ein Zwischenraum vorhanden ist.

Das hat den Vorteil, daß an dem Sichtkörper durch dessen Doppelwandigkeit ein Isolierglaseffekt gegen das Beschlagen des Sichtkörpers ausgenutzt werden kann. Dieser Isolierglaseffekt, der bereits von doppelwandigen Fensterscheiben bekannt ist, unterstützt auch die Wirkung der Beheizung des Sichtkörpers.

Eine bevorzugte Ausführung der Erfindung sieht vor, daß der Zwischenraum zwischen den einander gegenüberliegenden Oberflächen des Sichtkörpers und der Heizeinrichtung durch mindestens einen Distanzkörper vorgegeben wird.

Das hat den Vorteil, daß zwischen Sichtkörper und Heizeinrichtung ein definierter Zwischenraum entsteht.

Eine weitere bevorzugte Ausführung der Erfindung sieht vor, daß die Befestigungsmittel mit denen die Heizeinrichtung an dem Sichtkörper angebracht ist gleichzeitig die Aufgabe von Distanzkörpern übernehmen. Vorteilhafterweise sind so keine oder nur eine geringe Anzahl weiterer Distanzkörper nötig.

Bei einer weiteren bevorzugten Ausführung der Erfindung ist wenigstens über Teilbereiche des Außenumfangs des Heizkörpers mindestens ein weiterer Distanzkörper zwischen den einander gegenüberliegenden Oberflächen des Sichtkörpers und der Heizeinrichtung vorhanden. Dieser kann wenigstens über Teilbereiche von diesen eine Abdichtung bilden. So wird vorteilhafterweise der Isolierglaseffekt verstärkt und zwischen Sichtkörper und Heizeinrichtung kann kein Schmutz eindringen.

Bei einer weiteren vorteilhaften Ausführung der Erfindung ist das Befestigungsmittel als elektrischer Steckkontakt ausgebildet und elektrisch leitend mit der Heizeinrichtung verbunden. Das hat den Vorteil, daß für den elektrischen Kontakt zu einer Stromquelle keine weiteren Einrichtungen am Sichtkörper nötig sind.

Wenn die Heizeinrichtung auf der einem Benutzer zugewandten Seite des Sichtkörpers befestigt ist und der Distanzkörper in eine Bohrung in dem Sichtkörper hinein ragt, kann vorteilhafterweise von außen, der dem Benutzer abgewandten Seite, durch eine Steckeinrichtung eine elektrische Spannung an den Heizkörper angelegt werden.

Die Heizeinrichtung kann sowohl aus planer Plattenware, als auch mittels Spritzguß hergestellt werden. Besteht die Heizeinrichtung aus planem, biegsamem Plattenmaterial, so hat das den Vorteil, daß sie einfach sowohl für plane, aus Plattenware gefertigte Sichtkörper, als auch für dreidimensional geformte Sichtkörper verwendet werden kann. Besitzt die Heizeinrichtung dagegen eine dreidimensionale, unveränderbare Form, die diese insbesondere durch Spritzgießen oder thermische Verformung erhält, so ist sie besonders gut verwendbar sowohl für dreidimensional geformte, ebenfalls spritzgegossene oder thermogeformte Sichtkörper, als auch für aus planer Plattenware gefertigte, zwei- oder dreidimensional verformte Sichtkörper.

Zwei bevorzugte Ausführungsbeispiele der Erfindung sind in der nachfolgenden Beschreibung und der zugehörigen Zeichnung näher dargestellt. Es zeigen:
- Figur 1: eine Frontansicht eines schematisch gezeichneten Visiers eines Schutzhelms für Motorradfahrer,
- Figur 2: eine weitere mögliche Ausführungsform des Visiers für einen Schutzhelm für Motorradfahrer und
- Figur 3: einen Querschnitt durch einen Teilbereich eines Visiers entsprechend Figur 1 oder Figur 2, im Bereich eines elektrischen Steckkontakts.

Eine zuverlässige Antibeschlagwirkung, zum Beispiel bei einer Visierscheibe 1 eines nicht gezeichneten Schutzhelms für einen Motorradfahrer, ist durch flächige Beheizung der Visierscheibe 1 im Sichtfeld des Motorradfahrers möglich. Eine solche Beheizung kann zum Beispiel über Heizdrähte, eine transparent leitfähige Beschichtung oder aufgedruckte Leiterbahnen erfolgen.

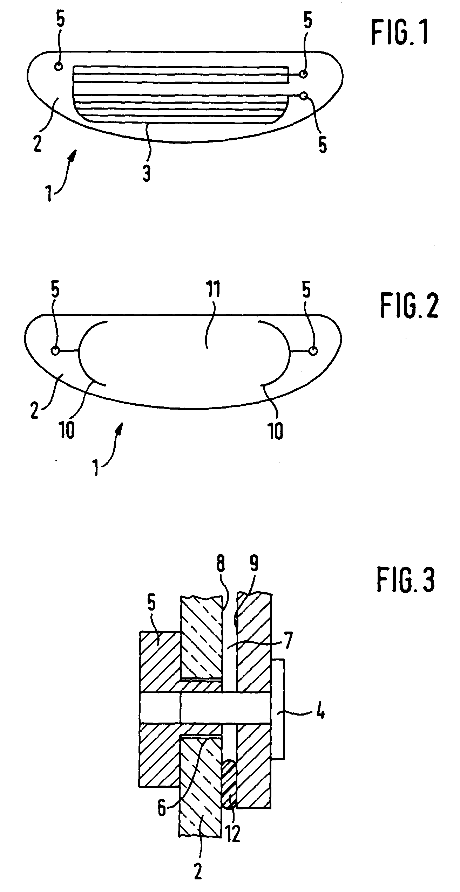

So zeigt Figur 1 eine Visierscheibe 1, bestehend aus einem Sichtkörper 2, mit einer aus elektrisch leitfähigem und durchsichtigem Material bestehenden Heizeinrichtung, unter Verwendung von Heizdrähten 3, die lösbar am Sichtkörper 2 angebracht ist. Die Heizeinrichtung befindet sich, in einer Richtung auf den Kopf des Schutzhelmträgers zu gesehen, hinter dem Sichtkörper 2 und ist über ein Befestigungsmittel, das als Distanzkörper 4 (Figur 3) dient, so am Sichtkörper 2 angebracht, daß zwischen den einander gegenüberliegenden Oberflächen 8, 9 des Sichtkörpers 2 und der Heizeinrichtung ein Zwischenraum 7 vorhanden ist. Es können auch mehrere Befestigungsmittel als Distanzkörper 4 dienen, mit denen die Heizeinrichtung an dem Sichtkörper 2 befestigt ist. Diese Befestigung kann zum Beispiel folgendermaßen ausgeführt sein: Der Sichtkörper 2 besitzt Bohrungen 6, in die die Distanzkörper 4, in diese hineinragend, eingeschoben und dort über ein Zwischenstück 5 gesichert sind. Der in den Sichtkörper 2 hineinragende Distanzkörper 4 dient als Befestigungsmittel der Heizeinrichtung am Sichtkörper 2 und zum Vorgeben des Zwischenraums 7. Außerdem ist der Distanzkörper 4 hier zusätzlich noch zusammen mit dem Zwischenstück 5 als elektrischer Steckkontakt ausgebildet, so daß darüber die Heizdrähte 3 mit elektrischem Strom zum Aufheizen der Heizeinrichtung versorgt werden können.

Figur 2 zeigt eine Visierscheibe 1, bestehend aus Sichtkörper 2 und Heizeinrichtung, mit Distanzkörpern 4, die zusammen mit Zwischenstücken 5 als elektrische Steckkontakte ausgebildet sind, in anderer Ausführungsform. Die Heizeinrichtung besteht aus Elektroden 10 und einer zwischen diesen liegenden transparenten, elektrisch leitfähigen Beschichtung 11.

Figur 3 zeigt den Aufbau einer Distanzkörperverbindung zwischen Sichtkörper 2 und Heizeinrichtung. Zwischen Sichtkörper 2 und Heizeinrichtung besteht an den aneinander gegenüberliegenden Oberflächen 8, 9 ein Zwischenraum 7. Dieser Zwischenraum 7 wird aufrecht erhalten durch einen Distanzkörper 4 und zusätzlich noch durch eine Abdichtung 12 zwischen Sichtkörper 2 und Heizeinrichtung im Bereich des Außenumfangs der Heizeinrichtung. Der Distanzkörper 4 bildet mit dem Zwischenstück 5 eine elektrische Steckdose, die durch die Heizeinrichtung hindurch ragt und mit deren Elektroden 10 verbunden ist. Der Sichtkörper 2 besitzt eine Bohrung 6, in die das Ende des Distanzkörpers 4 hineinragt, das von der anderen Seite des Sichtkörpers 2 her durch das Zwischenstück 5 gesichert ist. So dienen die trennbaren elektrischen Kontakte als Befestigungsmittel der Heizeinrichtung am Sichtkörper 2 und zum Vorgeben des Zwischenraums 5. Sie können somit, je nach Wahl des Werkstoffs und/oder des Fertigungsverfahrens des Sichtkörpers 2 und/oder der Heizeinrichtung, auch zur Formgebung von Sichtkörper 2 und/oder Heizeinrichtung beitragen, indem beide gegeneinander durch die elektrischen Kontakte verspannt werden.

## Patentansprüche

1. Sichtkörper (2) für einen Schutzhelm oder eine Schutzbrille mit einer aus elektrisch leitfähigem und mindestens teilweise durchsichtigem Material bestehenden Heizeinrichtung, die lösbar am Sichtkörper (2) angebracht ist, dadurch gekennzeichnet, daß zwischen einander gegenüberliegenden Oberflächen (8,9) des Sichtkörpers (2) und der Heizeinrichtung, wenigstens über Teilbereiche von diesen ein Zwischenraum (7) vorhanden ist.

2. Sichtkörper (2) nach Anspruch 1, dadurch gekennzeichnet, daß der Zwischenraum (7) zwischen den einander gegenüberliegenden Oberflächen (8,9) des Sichtkörpers (2) und der Heizeinrichtung durch mindestens einen Distanzkörper (4) vorgegeben wird.

3. Sichtkörper (2) nach Anspruch 2, dadurch gekennzeichnet, daß der Distanzkörper (4) ein Befestigungsmittel ist, mit dem die Heizeinrichtung an dem Sichtkörper (2) befestigt ist.

4. Sichtkörper (2) nach Anspruch 2 oder 3, dadurch gekennzeichnet, daß wenigstens über Teilbereiche des Außenumfangs des Heizkörpers mindestens ein weiterer Distanzkörper (12) zwischen den einander gegenüberliegenden Oberflächen (8,9) des Sichtkörpers (2) und der Heizeinrichtung vorhanden ist.

5. Sichtkörper (2) nach Anspruch 4, dadurch gekennzeichnet, daß der weitere Distanzkörper (12) zwischen den einander gegenüberliegenden Oberflächen (8,9) des Sichtkörpers (2) und der Heizeinrichtung wenigstens über Teilbereiche von diesen eine Abdichtung bildet.

6. Sichtkörper (2) nach einem der Ansprüche 2 bis 5, dadurch gekennzeichnet, daß der Distanzkörper (4) als elektrischer Steckkontakt ausgebildet und elektrisch leitend mit der Heizeinrichtung verbunden ist.

7. Sichtkörper (2) nach einem der Ansprüche 2 bis 6, dadurch gekennzeichnet, daß die Heizeinrichtung auf der einem Benutzer zugewandten Seite des Sichtkörpers (2) befestigt ist und der Distanzkörper (4) durch eine Bohrung (6) in den Sichtkörper (2) hinein ragt.

8. Sichtkörper (2) nach einem der Ansprüche 1 bis 7, dadurch gekennzeichnet, daß die Heizeinrichtung aus biegsamem und/oder thermisch verformtem Plattenmaterial besteht.

9. Sichtkörper (2) nach einem der Ansprüche 1 bis 8, dadurch gekennzeichnet, daß die insbesondere durch Spritzgießen hergestellte Heizeinrichtung eine dreidimensionale, unveränderbare Form besitzt.
